# EUROPEAN PATENT APPLICATION

(11) **EP 4 579 604 A1**
(43) Date of publication of application: **02.07.2025**
(21) Application number: 23383385.4
(22) Date of filing: 27.12.2023
(51) Int. Cl.: G06V 10/25, G06V 10/20, G06V 10/26, G06V 10/44, G06V 10/62, G06V 10/75, G06V 10/80, G06V 20/69, G06V 20/64, G01N 15/10, G01N 15/1434

(54) **A METHOD, A COMPUTER PROGRAM AND A SYSTEM FOR SPERM QUALITY DETERMINATION**

(71) Applicant: Fundació Institut de Ciències Fotòniques, 08860 Castelldefels (ES)
(72) Inventor: CASTRO OLVERA, Gustavo, 08860 CASTELLDEFELS (ES); VALDÉS E., Claudia P., 08860 CASTELLDEFELS (ES); ANDILLA, Jordi, 08860 CASTELLDEFELS (ES); LOZA ALVAREZ, Pablo, 08860 CASTELLDEFELS (ES)
(74) Representative: Ponti & Partners, S.L.P

(57) **Abstract**

The present invention relates to a method for sperm quality determination, comprising:
a) locating and tracking image data of a spermatozoon in a plurality of sequentially acquired volumetric groups of images of a sample that includes a plurality of freely swimming spermatozoa, wherein the sequential acquisition has been carried out by means of non-point scanning optical sectioning microscopy;
b) extracting features from the image data, the extracted features being three-dimensional morphological and dynamics features of the head and flagellum of the spermatozoon;
c) determining a quality score for the spermatozoon based on the extracted three-dimensional features, applying statistics, and conducting a comparison with benchmarks referring to spermatozoa morphology and dynamics; and
d) providing the quality score.

The present invention also relates to a computer program and a system implementing the method of the invention.

## Description

### FIELD OF THE INVENTION

The present invention generally relates, in a first aspect, to a method for sperm quality determination, using statistics and extracted three-dimensional morphological and dynamics features of the head and flagellum of the spermatozoa.

A second aspect of the present invention relates to a computer program adapted to implement the method of the first aspect of the invention.

A third aspect of the present invention relates to a system adapted to implement the method of the first aspect of the invention.

### BACKGROUND OF THE INVENTION

There are different proposals in the prior art aimed to determine the quality of sperm and/or of selecting/sorting that sperm.

For sperm selection, there are proposals based on microfluidics, such as that disclosed in patent EP3071704B1, where selection of the sperm is based on the capability of sperm passing through microchannels, which thus form obstacles not allowing the sperm to freely swim, and are not capable of 3D imaging, hence they do not use the 3D features to select the spermatozoa.

Also for sperm selection, there are systems, such as that described in patent EP1054948B1, which are based on digital microscopy known as computer-assisted sperm analysis (CASA). In those systems, the spermatozoa are placed in a physiological chamber and then a digital camera is used into a conventional microscope for image acquisition over time. The obtained images are then post-processed via the CASA software. This software detects the heads of the sperms and tracks their position, providing valuable information like the 2D trajectories of motile sperm and quantify their motility. Typical CASA systems strongly constrain the spermatozoa movement in a 2D plane, confining the sample to 20µm in one of the dimensions. The spermatozoa are not freely moving in a volume, and the analysis is limited to 2D motion.

There are also proposals based on piezoelectric systems, which allow tracking sperm in three dimensions, such as that described in G. Corkidi, et al. "Tracking sperm in three-dimensions," Biochemical and Biophysical Research Communications, vol. 373, no. 1, pp. 125-129, Aug. 2008, or in H. Gadêlha et al., "Human sperm uses asymmetric and anisotropic flagellar controls to regulate swimming symmetry and cell steering," Sci. Adv., vol. 6, no. 31, p. eaba5168, Jul. 2020.

In those piezoelectric proposals, to scan the volume of the sperm sample, a piezoelectric stage is used to rapidly adjust the position of a microscope objective. The magnification of the objective defines the field of view and the depth of field of the technique. A low magnification objective enables a large field of view and imaging of multiple spermatozoa, but with limited resolution that only allows head tracking. In contrast, a high magnification objective provides enhanced resolution for recovering morphological details of the head and flagellum, at the expense of a reduced field of view that allows only a few spermatozoa to be visualized. Therefore, there is a trade-off between high resolution to resolve morphology and dynamics in 3D, and the number of sperms that can be resolved.

Therefore, in the piezoelectric proposals, the characterisation is restricted to few sperm at a time, limited by the field of view of the microscope objective (60x, NA=1).

There are also proposals for 3D fast tracking of human sperms, such as that disclosed in T. W. Su et al, "High-throughput lensfree 3D tracking of human sperms reveals rare statistics of helical trajectories," Proc. Natl. Acad. Sci. U.S.A., vol. 109, no. 40, pp. 16018-16022, Oct. 2012, where a stereoscopic approach for localizing and tracking multiple spermatozoa in 3D is employed.

In a subsequent study by the same group, M. U. Daloglu et al. "Label-free 3D computational imaging of spermatozoon locomotion, head spin and flagellum beating over a large volume", Light: Science & Applications vol 7, no. 1, pp. 17121, Jan. 2018, a more sophisticated computational analysis was utilized to extract the 3D head motion and spin, as well as the flagellar beating patterns of the spermatozoa. Nonetheless, the optical method employed lacks the resolution capacity to reconstruct morphological details of the head or flagellum.

There are also proposals based on interferometric systems, such as that described in Gili Dardikman-Yoffe et al, "High-resolution 4-D acquisition of freely swimming human sperm cells without staining," Sci. Adv., vol. 6, no. 15, p. eaay7619, Apr. 2020.

The technique utilized in that study employs an interferometric setup to measure phase changes induced by refractive index differences throughout the entire volume. This provides high-resolution details of the 3D morphology and dynamics of the sperm over time. However, the method is restricted to non-overlapping spermatozoa in the beam propagation direction.

Therefore, in the interferometric proposals, the characterisation is restricted to few sperm at a time (<10). If there is overlapping of spermatozoa in the field of view, i.e. shadowing, this method fails due of lack of optical sectioning.

It is, therefore, necessary to provide an alternative to the state of the art by providing a method/system which do not possess the above mentioned drawbacks of those of the prior art.

### SUMMARY OF THE INVENTION

To that end, the present invention relates, in a first aspect, to a method for sperm quality determination, comprising the computer-implemented steps of:
a) locating and tracking image data representative of at least one spermatozoon in a plurality of sequentially acquired volumetric groups of images of a sample that includes a plurality of freely swimming spermatozoa, and wherein the sequential acquisition has been carried out by means of non-point scanning optical sectioning microscopy;
b) extracting features from that image data, the extracted features being three-dimensional morphological and dynamics features of the head and flagellum of the at least one spermatozoon;
c) determining a quality score for the at least one spermatozoon based on said extracted three-dimensional features, for at least part of the extracted three-dimensional dynamics features after applying statistics thereon or on parameters derived therefrom, and on a comparison with benchmarks referring to spermatozoa morphology and dynamics; and
d) providing the quality score.

Recently, defects in the spermatozoa flagellum have been associated to be one of the causes that could lead to fertilization failure (Inaba, K. and Mizuno, K. (2016), Sperm dysfunction and ciliopathy. Reprod. Med. Biol., 15: 77-94. https://doi.org/10.1007/s12522-015-0225-5). This makes the study of the flagellum morphology and dynamics relevant in the evaluation procedures before an in-vitro fertilization (IVF). Moreover, it shows the necessity of sperm evaluation systems that allow simultaneous observation of the head morphology, movements and as well as the full recording of the flagellum dynamics. The latter would need a system capable of fast imaging, in 3D, of flagellum's fast oscillation of freely moving sperms over time. Given the statistical nature of some parameters relevant in the evaluation of the sperm quality, it is also required that the system allows simultaneous imaging of a large number of sperms. All these requirements are met by the method of the first aspect of the present invention, and also by the second and third aspects of the present invention, which will be described below.

The method of the first aspect of the invention allows simultaneous imaging of multiple freely moving sperms under low to high number/density of sperm, under natural or controlled environmental conditions. This is possible due to the depth of field decoupling from the detection objective through non-point scanning optical sectioning.

In the present invention, the term score must be interpreted in a broad manner, so that it refers to any type of element indicating the quality of the sperm, whether numeric, graphic, textual, visual, audible, etc., while step d) comprises providing the quality score in any possible manner, whether to a user, by means of an appropriate device (display, speaker, haptic device, etc.) and/or to a system/machine, such as an automatic sperm selector using as input the received quality score.

For some embodiments, step b) comprises extracting part or all of the three-dimensional morphological and dynamics features from 2D image data, i.e. without performing a 3D reconstruction, while for other embodiments step b) comprises extracting part or all of the three-dimensional morphological and dynamics features from 3D image data obtained from a 3D reconstruction.

In an embodiment, the statistics of step c) are applied only to part or all of the extracted three-dimensional dynamics features, such as to the dynamic feature(s) referring to the displacement of one or more points of the tail of the spermatozoon, or on parameters derived therefrom, such as the motility associated to that displacement(s). In this case, the results of those statistics are compared with benchmarks referring to spermatozoa dynamics, and the extracted three-dimensional morphological features are compared with benchmarks referring to spermatozoa morphology. Optionally, the extracted three-dimensional features not being object of those statistics are also compared with benchmarks referring to spermatozoa morphology and/or dynamics.

For an embodiment, step c) comprises determining the quality score for the at least one spermatozoon based on statistics performed also on at least part of the extracted three-dimensional morphological features, such as to the morphological feature(s) referring to the length of the head of the spermatozoon, or on parameters derived therefrom, such as the ellipticity of that head. In this case, the results of those statistics are compared with benchmarks referring to spermatozoa morphology and dynamics. Optionally, the extracted three-dimensional features not being object of those statistics are also compared with benchmarks referring to spermatozoa morphology and/or dynamics.

According to an embodiment, the quality score of steps c) and d) is an individual quality score for the at least one spermatozoon.

For an implementation of that embodiment, steps a) and b) are applied to at least two spermatozoa, steps c) and d) respectively comprising determining and providing at least two respective individual health scores for the at least two spermatozoa.

Therefore, individual statistics for the spermatozoa may be performed for embodiments of the present invention, ad used to determine individual quality scores.

For another embodiment, steps a) and b) are applied to at least two spermatozoa, the quality score of steps c) and d) being a global quality score, wherein step c) comprises determining that global quality score based on the extracted three-dimensional features of the at least two spermatozoa, for at least part of the extracted three-dimensional dynamics features of the at least two spermatozoa after applying statistics thereon or on parameters derived therefrom, and on a comparison with benchmarks referring to spermatozoa morphology and dynamics.

For an implementation of that embodiment, step c) comprises determining the global quality score for the at least two spermatozoa based on statistics performed also on at least part of the extracted three-dimensional morphological features of the at least two spermatozoa, such as to the morphological feature(s) referring to the length of the head of the spermatozoa, or on parameters derived therefrom, such as the ellipticity of that head. In this case, the results of those statistics are compared with benchmarks referring to spermatozoa morphology and dynamics. Optionally, the extracted three-dimensional features not being object of those statistics are also compared with benchmarks referring to spermatozoa morphology and/or dynamics.

According to an embodiment, step b) comprises:
b1) for the three-dimensional morphological features:
   b1a) identifying in the image data the head and tail of the at least one spermatozoon; and
   b1b) extracting the following three-dimensional morphological features: head length, head width, head thickness, and tail length of the at least one spermatozoon;
b2) for the three-dimensional dynamics features:
   b2a) identifying in the image data the cartesian coordinates in each time in three dimensions of the centroid of the head and different points along the tail of the at least one spermatozoon; and
   b2b) extracting the following three-dimensional dynamics features: a total forward swimming speed of the at least one spermatozoon and average tail displacements of those tail different points relative to a centre of movement, and deriving a motility parameter therefrom.

For an embodiment:
- sub-step b1a) further comprises identifying in the image data also the midpiece of the at least one spermatozoon;
- sub-step b1b) further comprises extracting the following three-dimensional morphological feature: midpiece length of the at least one spermatozoon;
- sub-step b2a) further comprises identifying in the image data the cartesian coordinates in each time in three dimensions of the centroid of the midpiece of the at least one spermatozoon; and
- sub-step b2b) further comprises extracting the following three-dimensional dynamics feature: average midpiece displacements of the midpiece relative to a centre of movement, and deriving said motility parameter also based on said average midpiece displacements.

For another embodiment:
- sub-step b1b) further comprises deriving a spermatozoon head ellipticity parameter from the extracted head length, head width, and head thickness features; and/or
- sub-step b2b) further comprises deriving the following parameters: pitch, roll and yaw angles, based on the cartesian coordinates in each time in three dimensions of the centroid of the head of the at least one spermatozoon.

In addition to the above described computer-implemented steps, for some embodiments, the method of the first aspect of the present invention further comprising, optically and in vitro, the step of sequentially acquiring the above mentioned plurality of volumetric groups of images of a sample that includes a plurality of freely swimming spermatozoa, wherein that sequential acquisition is carried out by means of non-point scanning optical sectioning microscopy.

That sequential acquisition step is performed so that due to the field of view of the microscopy used and to the density of spermatozoa in the sample, each of the acquired images of each volumetric group includes a plurality of spermatozoa, i.e. simultaneous imaging of multiple freely moving sperms can be performed, also due to the depth of field decoupling from the detection objective through non-point scanning optical sectioning.

For an embodiment, the method further comprises providing that sample, in vitro, within a three-dimensional chamber allowing the plurality of spermatozoa freely swim, with the appropriate density and arranged at the right place within the above mentioned field of view, so that the above conditions are met, and thus each of the acquired images of each volumetric group includes a plurality of spermatozoa.

According to an implementation of that embodiment, the method comprises performing the step of sequentially acquiring the volumetric groups of images at a minimum volumetric recording rate above 10 Hz, and acquiring, for each volumetric group, at least three images for three respective consecutive optical sections of the sample, although preferably at least ten images for ten respective consecutive optical sections of the sample are acquired, and, for some embodiments, even more, up to 3000 images per volumetric group.

For an embodiment for which the spermatozoa are freely swimming under natural conditions (i.e. with no temperature modification, or other controlled environmental conditions modifications, no hormones or chemicals used for slowing their movement, etc.), so that their tail beat frequency oscillate around 35 Hz, the method comprising performing the step of sequentially acquiring said volumetric groups of images at a minimum volumetric recording rate above 70 Hz, i.e. doubling that tail beat frequency, following the Nyquist criterion.

For an embodiment, the non-point scanning optical sectioning microscopy is a structured illumination microscopy (SIM, 3D-SIM), and thus the images of consecutive optical sections of the sample come from the information contained in the light emitted or scattered by the elements of the sample present at those optical sections when a structured pattern is created in the sample by means of non-uniform illumination. Typically, multiple raw images with a phase shift in the illumination pattern are used to reconstruct an optically sectioned image, although any other kind of structured illumination microscopy using other types of illumination patterns can also be used according to the present invention.

For another embodiment, the non-point scanning optical sectioning microscopy is a light sheet microscopy (LSM), and thus the images of consecutive optical sections of the sample are acquired at consecutive image planes, at each image plane a light sheet being consecutively arranged to illuminate the sample and then the light emitted by the illuminated parts of the sample are picked up by an imaging lens and detected by a detector to form an image.

For different implementations of that embodiment, to increase the resolution to resolve sperm morphology provided by the optical sectioning of LSM, the technique is complemented with additional approaches to extend the depth of field, such as wavefront coding (WFC) and/or with electrotuneable lenses (ETL) and/or tuneable acoustic-gradient index refraction lenses, to compensate for a possible defocusing which could be introduced by the motion of the light sheet, if that was the case.

For an embodiment of the method of the first aspect of the invention, the method further comprises an automatic selection of the sperm based on the determined quality score(s).

With respect to the above mentioned benchmarks referring to spermatozoa morphology and dynamics, depending on the embodiment, they are just benchmarks provided based on scientific knowledge about morphology and dynamics of high quality sperm, or are dynamically obtained, for example by a deep or machine learning algorithm, on the image data obtained from the current sample or on image data of a previous sample that includes freely swimming spermatozoa having a high quality.

A second aspect of the present invention relates to a computer program, including code instructions that, when executed on at least one processor, implement the computer-implemented steps of the method of the first aspect of the present invention.

A computer program product is further provided by the present invention, comprising a tangible medium and, stored therein, the computer program of the second aspect of the present invention.

A third aspect of the present invention relates to a system for sperm quality determination, comprising a processor and a memory storing instructions that when executed by the processor cause the processor to implement the computer-implemented steps of the method of the first aspect of the present invention.

According to an embodiment, the system of the third aspect of the present invention, further comprises a non-point scanning optical sectioning microscope configured and arranged to implement the sequential acquisition of the plurality of volumetric groups of images of a sample that includes a plurality of freely swimming spermatozoa, and a three-dimensional chamber configured and arranged for in vitro housing said sample so that said plurality of spermatozoa can freely swim within said three-dimensional chamber.

The three-dimensional chamber is sized to allow the plurality of spermatozoa to swim freely, with the appropriate density, and arranged at the right place within the above mentioned field of view of the microscope, so that the conditions explained above are met, and thus each of the acquired images of each volumetric group includes a plurality of spermatozoa.

For an implementation of that embodiment, the non-point scanning optical sectioning microscope is a structured illumination microscope.

For an implementation of that embodiment, the non-point scanning optical sectioning microscope is a light sheet microscope (LSM).

For some variants of that implementation, the system further comprises additional mechanisms to extend the depth of field, such as wavefront coding (WFC) and/or electrotuneable lenses (ETL) and/or tuneable acoustic-gradient index refraction lenses, to compensate a possible defocusing which could be introduced by the motion of the light sheet, if that was the case.

For an embodiment, the system of the third aspect of the invention further comprises a device, such as a display, speaker, haptic device, etc., for providing the quality score(s) to a user.

For an embodiment, the system of the third aspect of the invention further comprises automatic selector with an input for the received the quality score(s), and which is configured to automatically perform the sperm selection based on the received quality score(s).

Since according to the present invention, no obstacles interfering the trajectories of the spermatozoa in the FOV are present, every spermatozoon swimming in any direction can be considered in the statistics, even in samples with low sperm concentration. Then, since the present invention allows the natural swimming of sperm, the data obtained from the 3D dynamic are more reliable than in those methods/systems not allowing that free swimming.

The present invention relies on sperm statistics based on 3D morphology and/or dynamics, and is capable of simultaneous characterisation of many sperm in a large FOV, hence, reducing the acquisition time to have enough data for meaningful statistics.

The present invention can be applied to determine the quality of human sperm and of non-human sperm.

There are many application for the present invention, including the selection of high-quality sperms. Here the industry is divided into two categories:
- Human applications: In the reproduction clinics and in the pharmaceutical industry. Used for studies of the quality of the sperm; pharmacological treatments, contraceptive methods and assisted reproduction.
- Animal application: in the food industry, for ensuring the production of high quality meat, fish, milk, wool, etc; for reproduction of high quality farm animals. In veterinary clinics for the equine breeding etc.

### BRIEF DESCRIPTION OF THE FIGURES

In the following some preferred embodiments of the invention will be described with reference to the enclosed figures. They are provided only for illustration purposes without however limiting the scope of the invention. In accordance with common practice, the components in the figures are drawn to emphasize specific features and they are not drawn to the right scale.
Figure 1 is a flow chart showing an embodiment of the method of the first aspect of the invention.
Figure 2 schematically shows a 3D spermatozoon localization segmentation, according to the method of the first aspect of the present invention, for an embodiment.
Figure 3 schematically shown the identification of the position of the head and of five points along the tail of a spermatozoon, according to the method of the first aspect of the present invention, for an embodiment.
Figure 4 shows different images acquired at different consecutive times, according to the method of the first aspect of the present invention, for simultaneous tracking multiple spermatozoa over time, for an embodiment.
Figure 5 schematically shows the system of the third aspect of the present invention, for an embodiment.

### DESCRIPTION OF THE PREFERRED EMBODIMENTS

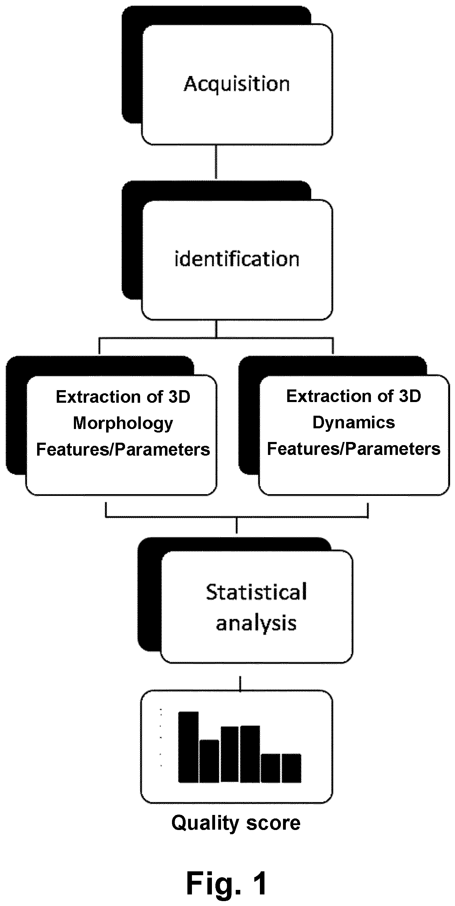

As generically shown in Figure 1, for an embodiment, the method of the first aspect of the present invention comprises:
- an acquisition step, comprising, optically and in vitro, sequentially acquiring volumetric groups of images of a sample that includes a plurality of freely swimming spermatozoa, wherein the sequential acquisition is carried out by means of non-point scanning optical sectioning microscopy,
- a step a), or identification step, comprising locating and tracking image data representative of spermatozoa in the plurality of sequentially acquired volumetric groups of images,
- a step b1), for extracting, from the image data, 3D morphological features/parameters of the head and flagellum of the spermatozoa;
- a step b2), for extracting, from the image data, 3D dynamics features/parameters of the head and flagellum of said at least one spermatozoon;
- a step c), including:
   - a statistical analysis applied on at least part of the extracted 3D dynamics features, and
   - the determination of a quality score based on the extracted three-dimensional features/parameters, including the result of the statistical analysis, and on a comparison with benchmarks referring to spermatozoa morphology and dynamics;
- a step d) comprising the provision of the quality score (the determination and provision of the quality score are represented by the same last block in Figure 1).

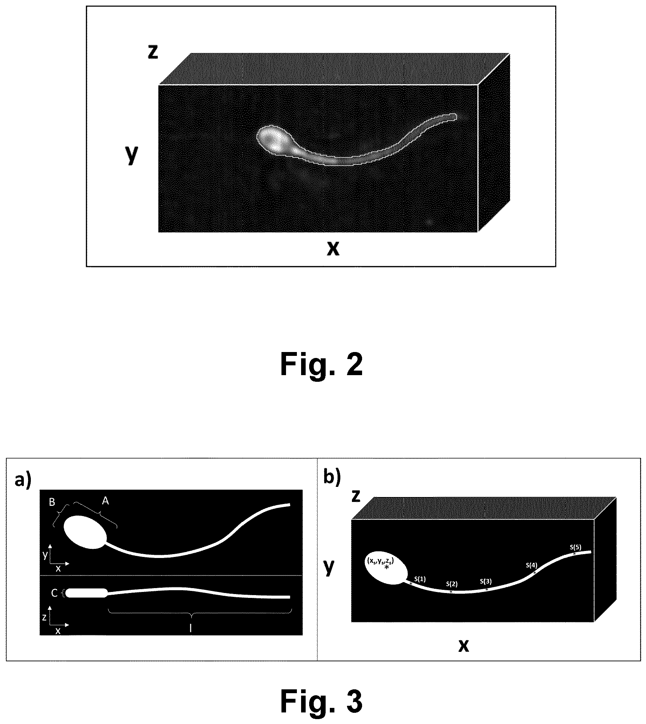

In the following, the description of the method of the first aspect of the present invention will be further detailed, for different embodiments, including the description of an experiment related to a specific implementation conducted by the present inventors, with reference to Figures 2, 3 and 4.

For that experiment, the present inventors used the LSM technique to probe the capabilities to resolve the 3D morphology and mobility/dynamics of the head and flagellum of the spermatozoa. Following the method of the present invention, volumetric images of the sperm are obtained. During the image processing, each sperm is located and segmented in 3D, as schematically shown in Figure 2.

The spermatozoa morphology involves the identification of the head, neck, and tail in each sperm. The 3D images allow the extraction of the head length A, width B, and thickness C, as well as the tail length I, as shown in Figure 3a.

For motility, the cartesian coordinates (x,y,z) in each time (t) in three dimensions for various parts of the spermatozoa are identified. That is, the centroid s(1) of the head (optionally also of the midpiece, not shown), as well as different points along the tail, particularly the five points s(1), s(2,) s(3), s(4), s(5) for the embodiment of Figure 3b.

The head centroid is identified as s(0), so with the values of (xs,ys,zs)=[x(0,t), y(0,t), z(0,t)] at each time point and with the A, B and C values, the dynamics of the head are described three-dimensionally. On the other hand, with the rest of the coordinates, i.e., of tail points s(1), s(2,) s(3), s(4), s(5), the dynamics of the spermatozoa tail are determined. The complete characterization of the 3D beat pattern on time is achievable by its curvature profile, κ(s,t), and torsion profile, τ(s,t).

Due to the complexity of the three-dimensional movement of the sperm there is still no convention on the name and definition of the relevant three-dimensional parameters in andrology to determine a healthy sperm. However, with the parameters A, B, C, I, x(s,t), y(s,t), z(s,t) extracted with the method of the first aspect of the invention, the parameters to characterize the morphology and motility of spermatozoa in the prior art could be obtained as well.

For an embodiment, the pitch, roll, and yaw angles are determined considering the centroid s(0) of the head (xs=0,y s=0,z s=0) as the new reference system for each spermatozoa. Likewise, the position of each point s(1), s(2,) s(3), s(4), s(5) in the tail [x(s,t), y(s,t), z(s,t)] are obtained.

On the other hand, for an embodiment, the parameter of the ellipticity of the head is measured directly in the method of the first aspect of the invention (i.e., derived from the extracted head length A, head width B, and head thickness C extracted features), in contrast to holographic and interferometric methods, where the retrieval of the parameters A, B, C is indirect since it requires the assumption of the ellipticity of the sperm head followed by the use of back propagation methods.

Below, Table 1 shows the minimum features/parameters necessary for the three-dimensional determination of spermatozoa morphology and mobility, for an embodiment, and which have been extracted (features) or determined (parameters) in the here explained experiment:

**Table 1**

| Morphology [um] | | | Motility | | |
|---|---|---|---|---|---|
| Head length | **= A** | 11.4±2.4 | Total speed [um/s] | 163±46 | |
| Head width | = **B** | 8.5±2.1 | Average tail point displacement relative to the centre of the movement [um] | s(1) | 2.7 |
| Head thickness | = **C** | 2.4±1.5 | | s(2) | 3.1 |
| Tail length | = **I** | 49 ± 6.2 | | S(3) | 3.6 |
| | | | | S(4) | 3.8 |
| | | | | S(5) | 6.4 |

For another embodiment, the length of the midpiece was also obtained: D = 4.1 ±3.7 and used as explained in a previous section, according to the method of the first aspect of the invention.

The present inventors note that under normal/natural conditions (i.e., no controlled environmental conditions), each spermatozoon has specific characteristics, requiring a specialized approach for a precise extraction of their three-dimensional information. These characteristics determine the optical specifications needed to ensure precise analysis.

The tail beat frequency of sperm typically oscillate around 35 Hz, requiring a minimum volumetric recording rate of 70 Hz (volumes/s) according to the Nyquist criterion. Additionally, the dimensions of the spermatozoa's head, particularly the smallest thickness is around 2um, Therefore the minimum optical resolution required for accurate visualization, is around 1 um.

On the other hand, to ascertain the free-swimming conditions of spermatozoon, the physical dimensions of the visualization container, i.e., the three-dimensional chamber Ch, must be at least double the size of the spermatozoon in all dimensions (i.e., 100x100x100 µm). However, if a sperm moves perpendicular to the detection axis, a minimum Depth of Field (DoF) of 10µm and a minimum Field of View (FoV) of 50µm × 10µm are required. In other words, the minimum observation volume needed to extract the three-dimensional features of a spermatozoon is 5000 µm³ (50×10×10µm). I.e., only part of the three-dimensional chamber Ch has to be focused in this volume of 5000 µm³. Moreover, the minimum number of f images of consecutive optical sections required to extract the three-dimensional characteristics of a sperm is three per volume.

The method of the first aspect of the present invention not only allows extracting three-dimensional parameters in normal sperm conditions, but is also useful for analyzing sperm that are outside the norm, either due to factors such as the health status of the donor, or due to alterations in physiological and/or environmental conditions, such as; temperature changes, presence of medications, among others. These conditions can alter both the morphology and dynamics of the spermatozoon, thus modifying the optical specifications needed to ensure precise analysis. Therefore, the non-point scanning optical sectioning allows decoupling the detection resolution from the depth of field, which allows multiple spermatozoa to be recorded simultaneously in three dimensions.

Then, as the present invention is also applicable when the spermatozoa are submitted to controlled physiological and/or environmental conditions, the ranges that should be considered are the ones included in Table 2 below:

**Table 2**

| | | |
|---|---|---|
| **Volumetric recording rate** | [volumes/s] | 10 to 1000 |
| **Optical resolution** | [µm] | 0.300 to 5 |
| **Field of View (FoV)** | [mm] | 0.100 to 10 |
| **Depth of Field (DoF)** | [mm] | 0.100 to 10 |
| **Frames per volume** | | 3 to 3000 |

In the following, the optical 3D sperm characterization conducted in the above described experiment, is described in a more detailed manner, for an embodiment, although alternative ways of conducting that characterization could be used, for other embodiments.

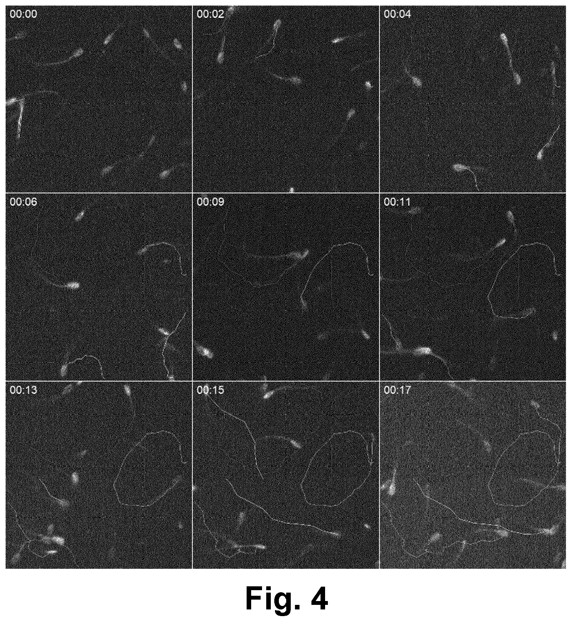

The present invention indeed allows simultaneous imaging of multiple freely swimming spermatozoa under high density conditions (i.e. ~200K sperms/mm³), defined by non-overlapping voxels of spermatozoon dimensions = 10×10×50µm, as shown in Figure 4, for images acquired at different consecutive times. It must be pointed out that Figure 4 only shows a particular optical section, for illustration purposes, but as explained above, the acquisition is made on volumes, i.e. a plurality of volumetric groups of images of corresponding consecutive optical sections are acquired.

For the present embodiment, each spermatozoon available in the imaged volume is segmented individually at each time point. Then, a bounding box is determined around each spermatozoon, so that it includes the whole cell (head and flagellum, and, optionally also the midpiece). Every box is then treated separately. The head is localized providing the spermatozoon position in 3D (x,y,z). Moreover, a 3D analysis of the head provides its dimensions (Sx, Sy, Sz), orientation (phi, psi, theta) and shape. Finally, the method also provides the flagellum length (L) and 3D curvature (C).

From the spermatozoa head's coordinates (x,y,z) the sperm motility parameters can be extracted: amplitude of lateral head displacement (ALH), beat cross frequency (BCF), curvilinear velocity (VCL), straight line velocity (VSL), average path velocity (VAP), linearity (LIN = VSL/VCL), and straightness (STR = VSL/VAP). In addition, the head dimension (Sx, Sy, Sz) and angular orientation (theta, phi, psi) provide morphological and motility information in 3D, as head rotation frequency. Furthermore, from the flagellum length (L) and curvature (C) it is possible to provide additional information/parameters, such as flagellum beating frequency, beating amplitude or oscillation modes.

From each volumetric time point, as indicated above, each spermatozoon was segmented individually and followed during its trajectory. With the parameters extracted from the position and angles of the head, as well as the curvature and size of the flagellum, the morphological and motor characteristics of each spermatozoon were determined. Table 3 shows the semen characteristics that can be extracted for healthy samples, for different embodiments.

**Table 3. Semen Characteristics and Sperm Morphology in healthy fresh samples**

| **2D features** | Literature |
|---|---|
| Normal morphology (%) | 23.1 ± 9.2 |
| Concentration (10⁶/mL) | 198.3 ± 108.8 |
| Motility (%) | 59.9 ± 16.5 |
| Motile sperm conc. (10⁶/mL) | 123.5 ± 81.2 |

| **Head sperm motion variables** | |
|---|---|
| ALH (µm) | 3.5 ± 0.8 |
| BCF (Hz) | 25.4 ± 3.4 |
| VCL (µm/s) | 86.2 ± 16.0 |
| VSL (µm/s) | 49.1 ± 9.2 |
| VAP (µm/s) | 64.0 ± 50.2 |
| Linearity (VSL/VCL) | 58.7 ± 6.7 |
| Straightness (VSL/VAP) | 81.4 ± 4.8 |
| Rapid (%) | 38.8 ± 17.8 |
| https://www.ncbi.nlm.nih.gov/pmc/articles/PMC3455361/pdf/10815_200 4_Article_295866.pdf | |

| **3D features** | |
|---|---|
| head (shape, position, rotation, wiggling) | |
| flagellum (length, curvature, beating frequency and amplitude, oscillation modes). | |

The 3D features/parameters could be reduced to the typical 2D motility parameters known in the art. Nevertheless, since in the present invention the features/parameters are obtained in a non-confined environment that allows the natural movement of the spermatozoa, they minimise the bias introduced by the 2D confinement of the prior art proposals. In addition, with the head dimension (Sx, Sy, Sz) and angular orientation (theta, phi, psi) provided with the method of the invention, morphological and motility information in 3D, as head rotation frequency can be obtained. Furthermore, from the flagellum length (L) and 3D curvature (C3D) it is possible to provide additional information, such as beating frequency, beating amplitude flagellum or oscillation modes.

The results presented here represent a partial proof of concept of the capabilities of the present invention to extract the individual spermatozoa information, under high-density (~200K sperms/mm3) condition, which will feed the data for statistical analysis, and then to generate and provide the quality score(s). The 3D information extracted with the method of the invention provides more information than the 2D methods. Moreover, since spermatozoa motion is not constrained in any direction (dimension), the results obtained are more reliable.

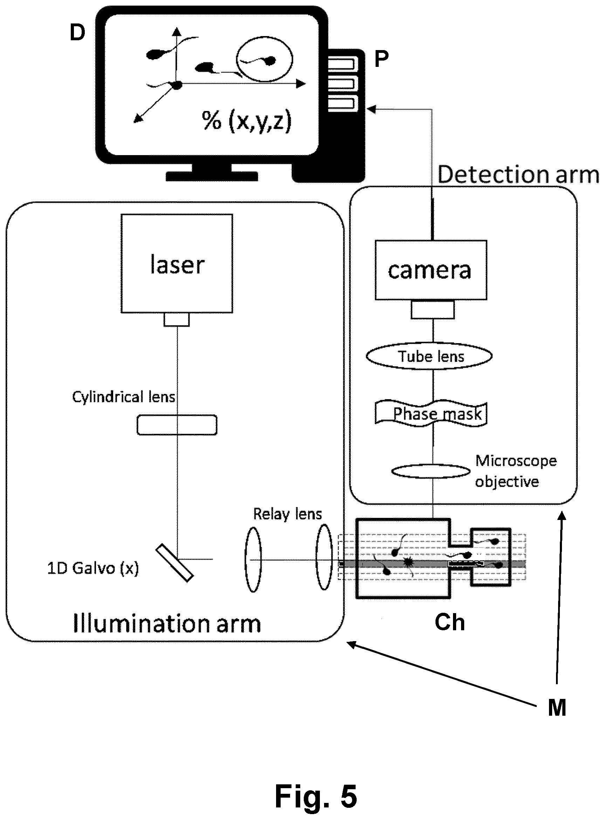

Figure 5 schematically shows the system of the third aspect of the present invention, for an embodiment for which the system comprises:
- a three-dimensional chamber Ch configured and arranged for in vitro housing a sample that includes a plurality of spermatozoa that can freely swim within the three-dimensional chamber Ch,
- a non-point scanning optical sectioning microscope M configured and arranged to implement the sequential acquisition of the plurality of volumetric groups of images of the sample,
- a computing entity P, comprising a processor and a memory storing instructions that when executed by the processor cause the processor to implement the computer-implemented steps of the method of the first aspect of the invention; and
- a display D for at least displaying the determined quality score, and in this case also for displaying a representation of the images of the spermatozoa.

For the illustrated embodiment, the microscope M is a light sheet microscopy (LSM), and comprises an illumination arm and a detection arm.

The illumination arm comprises a laser source, a cylindrical lens, a 1D galvo mirror, and a relay lens, to generate a light sheet and sequentially illuminate with the same different consecutive optical sections of the sample a plurality of times, each for a respective one of the plurality of volumetric groups.

The detection arm comprises a microscope objective, a phase mask for wavefront coding (WFC), a tube lens, and a camera, to pick up and detect the light emitted, through a wall of the 3D chamber Ch, by the illuminated parts of the sample at each optical section, and thus the images formed with that emitted light.

The detected images of the plurality of volumetric groups are provided to the computing entity C, which process the same as explained above, according to the method of the first aspect of the invention.

Specifically, the system used by the present inventors for conducting the above described experiment has the followings features: a laser (568 nm, Cobolt), a cylindrical lens (f=75mm, LJ1703RM-A, Thorlabs), a galvanometric mirror (GVS002, Thorlabs), a relay lens (AC254-150-A-ML and AC254-200-A-ML, Thorlabs) and an illumination lens (10x microscope objective, 0.30 NA, 3.5 mm WD, Nikon CFI Plan Fluorite) . A heating metal plate filled with water, which is in thermal contact with a FEP tube (2mm ID) which contains the spermatozoa, forms the physiological chamber. Illumination and collection lenses are immersed in water, in an iSPIM configuration. The detection is performed using a collection lens (20x microscope objective, 1 NA, 2 mm WD, Olympus), two achromatic lenses (AC508-200-A-ML, Thorlabs) a deformable mirror and a sCMOS camera (Hamamatsu Orca-Flash4.0 v3). Care was taken to place all the optical elements in the proper conjugated planes obtaining a total magnification of 22.2x.

And the system was used as described in the following to perform the above described experiment.

The signal was recorded for 10 min at a scanning speed of 80 volumes/second, every volume had a depth of 40 µm, containing 10 imaged planes. The size of each voxel was 0.3µm × 0.3µm × 4µm (x, y, z). After the acquisition, the collected data was structured in volumetric frames providing a sequence of 3D images. If necessary, (in the case of WFC, light field, Fourier light field) each volume was deconvolved with the corresponding 3D point spread function (PSF). Volumetric deconvolution was performed by deconvolving each frame with the PSF frame of the corresponding z (using the Richardson-Lucy-based algorithm). The volumetric PSF was generated from the distilling of the experimental PSF using the Zernike coefficients applied to the phase mask.

However, deconvolution can be done following classical deconvolution methods such as Richardson-Lucy, blind, Wiener algorithms or it can be done based on Machine learning, Simulated annealing, Genetic algorithms, among others. Likewise, the volumetric PSF necessary for the deconvolution can be measured directly in the experiment using a fluorescent bead (or an artificial star) or be generated from the simulation of the system, without it being necessary in cases like machine learning algorithms.

The schematic shown in Figure 5 can be varied in multiple manners, for implementing different kinds of LSMs or complement the same with further mechanisms, such as those intended to extend the depth of field, such as wavefront coding (WFC) (in addition or alternatively to the above mentioned phase mask), and/or electrotuneable lenses (ETL) and/or tuneable acoustic-gradient index refraction lenses, to compensate a possible defocusing which could be introduced by the motion of the light sheet, if that was the case.

Variation for other types of non-point scanning optical sectioning microscopes are also envisaged for the system of the third aspect of the invention.

A person skilled in the art could introduce changes and modifications in the embodiments described without departing from the scope of the invention as it is defined in the attached claims.

## Claims

1. A method for sperm quality determination, comprising the computer-implemented steps of:
a) locating and tracking image data representative of at least one spermatozoon in a plurality of sequentially acquired volumetric groups of images of a sample that includes a plurality of freely swimming spermatozoa, wherein said sequential acquisition has been carried out by means of non-point scanning optical sectioning microscopy;
b) extracting features from said image data, said extracted features being three-dimensional morphological and dynamics features of the head and flagellum of said at least one spermatozoon;
c) determining a quality score for the at least one spermatozoon based on said extracted three-dimensional features, for at least part of said extracted three-dimensional dynamics features after applying statistics thereon or on parameters derived therefrom, and on a comparison with benchmarks referring to spermatozoa morphology and dynamics; and
d) providing said quality score.

2. The method of claim 1, wherein step c) comprises determining said quality score for the at least one spermatozoon based on statistics performed also on at least part of said extracted three-dimensional morphological features or on parameters derived therefrom.

3. The method of claim 1 or 2, wherein said quality score of said steps c) and d) is an individual quality score for the at least one spermatozoon.

4. The method of claim 1 or 2, wherein said steps a) and b) are applied to at least two spermatozoa, said quality score of steps c) and d) being a global quality score, wherein step c) comprises determining said global quality score based on the extracted three-dimensional features of the at least two spermatozoa, for at least part of the extracted three-dimensional dynamics features of the at least two spermatozoa after applying statistics thereon or on parameters derived therefrom, and on a comparison with benchmarks referring to spermatozoa morphology and dynamics.

5. The method of any of the previous claims, wherein said step b) comprises:
b1) for the three-dimensional morphological features:
b1a) identifying in the image data the head and tail of the at least one spermatozoon; and
b1b) extracting the following three-dimensional morphological features: head length (A), head width (B), head thickness (C), and tail length (I) of the at least one spermatozoon;
b2) for the three-dimensional dynamics features:
b2a) identifying in the image data the cartesian coordinates (x,y,z) in each time (t) in three dimensions of the centroid (s(0) of the head and different points (s(1), s(2,) s(3), s(4), s(5)) along the tail of the at least one spermatozoon; and
b2b) extracting the following three-dimensional dynamics features: a total forward swimming speed of the at least one spermatozoon and average tail displacements of those tail different points (s(1), s(2,) s(3), s(4), s(5)) relative to a centre of movement, and deriving a motility parameter therefrom.

6. The method of claim 5, wherein:
- sub-step b1a) further comprises identifying in the image data also the midpiece of the at least one spermatozoon;
- sub-step b1b) further comprises extracting the following three-dimensional morphological feature: midpiece length (D) of the at least one spermatozoon;
- sub-step b2a) further comprises identifying in the image data the cartesian coordinates (x,y,z) in each time (t) in three dimensions of the centroid of the midpiece of the at least one spermatozoon; and
- sub-step b2b) further comprises extracting the following three-dimensional dynamics feature: average midpiece displacements of the midpiece relative to a centre of movement, and deriving said motility parameter also based on said average midpiece displacements.

7. The method of claim 5 or 6, wherein:
- sub-step b1b) further comprises deriving a spermatozoon head ellipticity parameter from the extracted head length (A), head width (B), and head thickness (C) features; and/or
- sub-step b2b) further comprises deriving the following parameters: pitch, roll and yaw angles, based on the cartesian coordinates (x,y,z) in each time (t) in three dimensions of the centroid of the head of the at least one spermatozoon.

8. The method of any of the previous claims, further comprising, optically and in vitro, the step of sequentially acquiring said plurality of volumetric groups of images of a sample that includes a plurality of freely swimming spermatozoa, wherein said sequential acquisition is carried out by means of non-point scanning optical sectioning microscopy

9. The method of claim 8, comprising performing the step of sequentially acquiring said volumetric groups of images at a minimum volumetric recording rate above 10 Hz, and acquiring, for each volumetric group, at least three images for three respective consecutive optical sections of the sample.

10. The method of claim 9, comprising performing the step of sequentially acquiring said volumetric groups of images at a minimum volumetric recording rate above 70 Hz.

11. The method of any of the previous claims, wherein said non-point scanning optical sectioning microscopy is a structured illumination microscopy or a light sheet microscopy.

12. A computer program, including code instructions that, when executed on at least one processor, implement the computer-implemented steps of the method of claims 1 to 11.

13. A system for sperm quality determination, comprising a processor and a memory storing instructions that when executed by the processor cause the processor to implement the computer-implemented steps of the method of claims 1 to 11.

14. The system of claim 13, further comprising a non-point scanning optical sectioning microscope (M) configured and arranged to implement the sequential acquisition of the plurality of volumetric groups of images of a sample that includes a plurality of freely swimming spermatozoa, and a three-dimensional chamber (Ch) configured and arranged for in vitro housing said sample so that said plurality of spermatozoa can freely swim within said three-dimensional chamber (Ch).

15. The system of claim 14, wherein said non-point scanning optical sectioning microscope (M) is a structured illumination microscope or a light sheet microscope.
